# FASCICULE DE BREVET EUROPEEN

(11) **EP 2 189 182 B1**
(45) Date de publication et mention de la délivrance du brevet: **08.06.2011**
(21) Numéro de dépôt: 09173825.2
(22) Date de dépôt: 22.10.2009
(51) Int. Cl.: A61N 1/37

(54) **Dispositif médical actif implantable comprenant des moyens de test de capture auriculaire**
Aktives medizinisches Implantat, das mit Testmitteln zur aurikulären Datenerfassung ausgestattet ist
Implantable active medical device comprising means for atrial capture testing

(30) Priorité: 19.11.2008 FR 0806461
(43) Date de publication de la demande: 26.05.2010
(73) Titulaire: ELA MEDICAL, 92541 Montrouge (FR)
(72) Inventeur: Casset, Cyrille, 33650, SAINT SELVE (FR)
(74) Mandataire: Dupuis-Latour, Dominique

(56) Documents cités:
- EP-A- 1 407 800
- EP-A- 1 433 497
- WO-A-95/19201
- WO-A-2005/089866
- US-A1- 2007 179 541

## Description

L'invention concerne les "dispositifs médicaux implantables actifs" tels que définis par la Directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle s'applique plus particulièrement à ceux de ces dispositifs qui possèdent au moins une sonde de détection/stimulation de l'oreillette ou des deux oreillettes. Il s'agit principalement des implants de type stimulateur ou défibrillateur simple chambre auriculaire, double chambre atrioventriculaire ou bien de type "multisite" triple ou quadruple chambre.

Après la stimulation de l'oreillette, il est important de recueillir l"'onde évoquée", c'est-à-dire l'onde de dépolarisation induite par la stimulation de l'oreillette, afin de déterminer si cette stimulation a été efficace ou non. Ce test ("test de capture") est notamment utilisé pour ajuster l'amplitude et/ou la largeur de l'impulsion de stimulation.

Dans le cas d'une stimulation auriculaire, la recherche de l'onde évoquée est particulièrement difficile.

En effet, l'onde évoquée présente non seulement une amplitude beaucoup plus faible que dans le cas du ventricule, mais elle est en outre beaucoup plus précoce : l'onde évoquée auriculaire (onde P) apparaît ainsi environ 10 ms après la stimulation de l'oreillette pour se terminer à environ 30 ms - alors que l'onde évoquée ventriculaire (onde R) est observée à environ 60 ms après la stimulation.

L'onde évoquée auriculaire, très précoce, se trouve en particulier mélangée, voire noyée, en sortie de l'amplificateur de détection dans le transitoire électrique appelé "réponse de l'amplificateur" consécutif à la stimulation électrique. Cette "réponse" est toujours présente, que l'onde évoquée soit présente ou non. Plus précisément, pour écouler les charges à l'interface coeur/électrode après la stimulation, on prévoit d'effectuer une décharge de l'énergie accumulée avec simultanément une déconnexion ou "blanking" des circuits de détection de l'amplificateur, typiquement pendant une durée de l'ordre de 14 ms. De plus, au moment de la reconnexion de l'amplificateur aux bornes de l'électrode à l'issue de la période de blanking apparaît en sortie de l'amplificateur un rebond transitoire de tension qui perdure quelques millisecondes jusqu'à ce que l'amplificateur soit complètement désaturé.

On comprendra que, dans ces conditions, il soit très difficile de détecter la présence d'une onde P évoquée, par exemple dans le cadre d'un test de capture auriculaire.

Le EP 1 433 497 A1 (ELA Medical) décrit une technique permettant d'améliorer la réponse de l'amplificateur de détection auriculaire par une inhibition contrôlée des circuits de détection auriculaire, de manière à rechercher des complexes spontanés post-stimulation après un "micro-blanking", le véritable "blanking" étant activé ultérieurement pour permettre la décharge complète de l'énergie accumulée à l'interface coeur/électrode après la stimulation.

Cette technique de mesure électrique directe des potentiels évoqués produits par l'oreillette implique un contrôle très précis de l'amplificateur de détection. Elle procure une amélioration certaine, mais ne garantit toutefois pas une détection sûre des complexes spontanés dans toutes les circonstances cliniquement envisageables, compte tenu de la variabilité des amplitudes, des instants de survenue, etc., des potentiels électriques que l'on cherche à mesurer pour détecter la présence ou l'absence de l'onde évoquée.

Le EP 1 407 800 A1 (ELA Medical) décrit une autre manière de détecter l'onde évoquée auriculaire, de façon indirecte, par analyse du rythme sinusal intrinsèque du patient et/ou des temps de conduction auriculo-ventriculaire du coeur du patient. Les méthodes indirectes restent toutefois limitées dans leurs possibilités d'application et peuvent être notamment mises en défaut par des événements cardiaques atypiques. Par exemple, une méthode consiste à stimuler l'oreillette et vérifier la présence de la conduction jusqu'au ventricule via une seconde sonde placée dans le ventricule du patient. Mais cette méthode ne peut pas être mise en oeuvre chez des patients présentant un bloc auriculo-ventriculaire complet (qui est, précisément, un trouble courant justifiant l'implantation d'une prothèse de type stimulateur) ; de façon plus générale, la fonction de test peut être leurrée par l'apparition de blocs auriculo-ventriculaires paroxystiques chez un patient ne présentant pas les symptômes d'un bloc complet.

Le WO 2005/089866 A1 propose une troisième approche, différente des deux précédentes (mesure électrique directe du potentiel de dépolarisation sur l'oreillette, ou détermination indirecte à partir de l'analyse du rythme et du séquencement atrial et ventriculaire), consistant à opérer une mesure de nature mécanique, en détectant la contraction de l'oreillette par un signal d'accélération endocardiaque délivré par un capteur accélérométrique approprié. Un tel capteur peut être présent sur la sonde auriculaire, ou sur une autre sonde possédant un tel capteur, placée soit directement dans l'oreillette, soit dans une autre position permettant de recueillir le signal d'accélération endocardiaque (signal EA) représentatif des contractions de l'oreillette.

Dans cette troisième approche le dispositif, après avoir stimulé l'oreillette, utilise un signal fonctionnel - le signal EA - représentatif de la mécanique cardiaque, à la place d'un signal issu de la propagation électrique de l'onde de dépolarisation. Ce signal mécanique peut également être exploité en complément du signal électrique, comme le US2007/0179541 A1 qui propose de mesurer et d'analyser le retard entre la détection électrique et la détection mécanique de la contraction atriale.

Le WO 2005/089866 A1 suggère d'utiliser le signal EA à diverses fins telles qu'optimisation du délai atrioventriculaire DAV dans le cas d'une stimulation double chambre, optimisation du délai interventriculaire DVV dans le cas d'une stimulation biventriculaire pour une thérapie de resynchronisation CRT, détection de capture dans une cavité cardiaque, etc.

Toutefois, comme pour le signal électrique, la composante du signal EA correspondant à l'activité de l'oreillette présente non seulement une amplitude beaucoup plus faible que dans le cas du ventricule, mais elle est en outre beaucoup plus précoce, ce qui rend sa détection et son analyse beaucoup plus difficile. C'est pour cette raison qu'il n'a jusqu'à présent pas été proposé de technique basée sur l'analyse de la composante atriale du signal EA qui procure des résultats réellement exploitables, malgré l'intérêt de disposer d'un signal reflétant directement l'activité mécanique du coeur.

L'un des buts de la présente invention est de remédier aux diverses difficultés exposées ci-dessus, en proposant une nouvelle technique de détection de l'onde évoquée auriculaire à partir du signal EA, qui tienne compte à la fois du caractère très précoce de cette dernière et de sa faible amplitude.

En particulier, cette technique peut être utilisée pour détecter les situations de perte de capture auriculaire, avérées par l'absence de contraction de l'oreillette après une stimulation auriculaire.

La détection d'une perte de capture auriculaire est en effet importante, car il est nécessaire dans un tel cas de prendre immédiatement des actions appropriées et/ou de modifier le fonctionnement de stimulateur, en particulier :
- application éventuelle d'une contre-stimulation auriculaire avec une énergie plus élevée ;
- augmentation de l'énergie des stimulations auriculaires suivantes ;
- dans le cas particulier des dispositifs équipés d'une commutation automatique du mode de fonctionnement, modification des critères de basculement éventuel en mode DDD tant que la capture auriculaire n'est pas rétablie.

Comme cela a été exposé plus haut, la présente invention se base sur l'approche consistant à détecter la contraction auriculaire par une analyse de l'accélération endocardiaque.

Diverses études cliniques ont été menées, qui montrent que l'accélération endocardiaque est un paramètre permettant de fournir des informations très complètes sur la mécanique cardiaque, aussi bien dans le cas d'un fonctionnement normal que d'un fonctionnement déficient. L'accélération endocardiaque, qui est mesurée par un accéléromètre couplé au muscle cardiaque, reflète en effet très précisément et en temps réel les phénomènes liés aux mouvements de la cavité cardiaque (la cavité auriculaire dans le cas de la présente invention).

Ainsi, le EP 0 515 319 A1 (Sorin Biomedica Cardio SpA) enseigne la manière de recueillir un signal EA au moyen d'une sonde endocavitaire intégrant un micro-accéléromètre permettant de mesure l'accélération endocardiaque.

On notera toutefois que, bien que dans la présente invention on fasse principalement référence à l'analyse d'un signal EA délivré par un capteur implanté (typiquement, un capteur placé sur une sonde endocavitaire) l'invention est également applicable à une analyse opérée à partir d'un signal EA externe obtenu de manière non invasive. Un tel signal EA peut être par exemple un signal issu d'un capteur fixé sur la poitrine du patient au niveau du sternum.

Ici, et dans la suite, lorsque l'on parlera de "signal EA", ce terme sera entendu comme couvrant aussi bien un signal EA externe, obtenu de manière non-invasive, qu'un signal EA endocavitaire, obtenu par un capteur d'accélération monté sur une sonde introduite dans une cavité du coeur du patient, ou encore un signal EA délivré par une sonde implantée épicardique en contact direct avec le myocarde.

L'invention propose un dispositif du type générique connu, par exemple d'après le WO 2005/089866 A1 précité, comportant les éléments énoncés dans le préambule de la revendication 1.

De façon caractéristique de l'invention, les moyens d'analyse du signal EA comprennent les éléments énoncés dans la partie caractérisante de la revendication 1. Les sous-revendications visent des mises en oeuvre subsidiaires, avantageuses.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre un exemple de signal d'accélération endocardiaque EA recueilli au cours de trois cycles cardiaques successifs.
La Figure 2 est une série de trois chronogrammes illustrant différents signaux caractérisant l'activité cardiaque au cours d'un cycle donné,
La Figure 3 est une série de quatre chronogrammes montrant la manière dont est opérée la détection de la composante représentative PEA4 selon un mode de réalisation préférentiel de l'invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par ELA Médical, Montrouge, France.

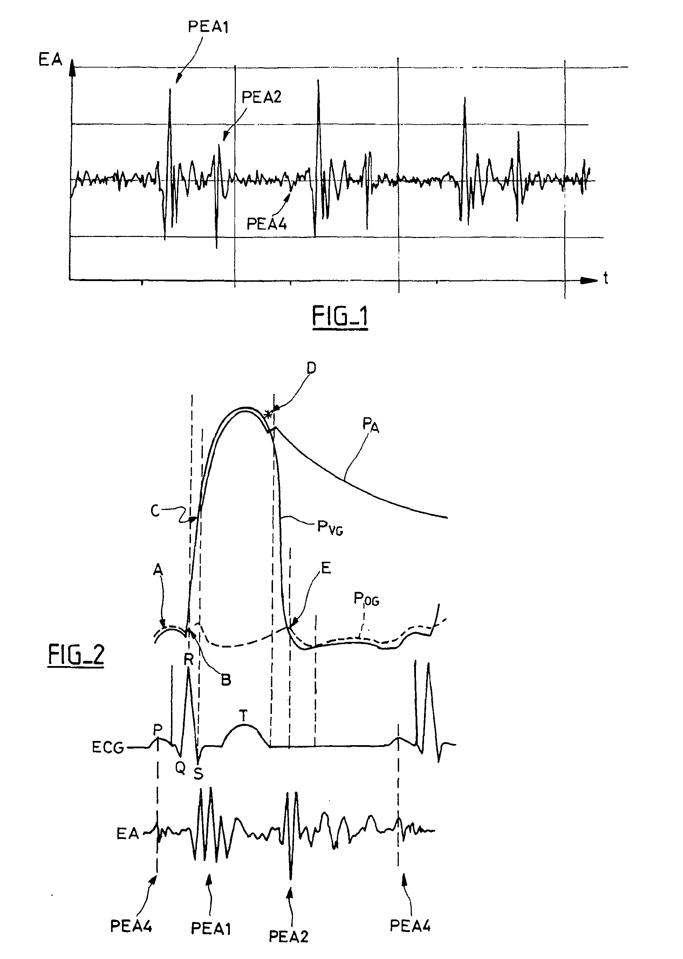

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. Comme illustré sur la Figure 1, qui est un exemple de signal d'accélération endocardiaque (EA) recueilli au cours de trois cycles cardiaques successifs, le signal EA forme au cours d'un cycle cardiaque deux pics principaux, correspondant aux deux bruits majeurs (sons S1 et S2 du phonocardiogramme) qu'il est possible de reconnaître dans chaque cycle d'un coeur sain :
- le premier pic d'accélération endocardiaque ("PEA1"), dont les variations sont étroitement liées aux variations de la pression dans le ventricule (l'amplitude du pic PEA1 étant, plus précisément, corrélée au maximum positif de la variation de pression dP/dt dans le ventricule gauche) ;
- le second pic d'accélération endocardiaque ("PEA2"), quant à lui, correspond à la phase de relaxation ventriculaire isovolumique et il est produit par la décélération brusque de la masse sanguine en mouvement dans l'aorte.

Les composantes EA1 et EA2 du signal EA sont celles qui correspondent aux deux pics d'accélération endocardiaque respectifs PEA1 et PEA2.

Le signal EA contient également deux autres composantes, d'amplitude très inférieure, appelées EA3 et EA4, correspondant aux sons S3 et S4 du phonocardiogramme.

L'invention s'intéresse plus particulièrement à la composante EA4, qui est directement liée à la présence d'une contraction auriculaire.

Essentiellement, l'invention se propose d'exploiter cette composante auriculaire afin de gérer les paramètres d'un stimulateur lié à l'activité auriculaire.

Cette composante présente en particulier un pic (ci-après "PEA4") qui, comme on peut le voir sur la Figure 1, se situe immédiatement avant le pic PEA1 - et pour cette raison ce pic est quelquefois désigné "PEA0" par les rythmologues, dans la mesure où, du point de vue électrique, la contraction de l'oreillette précède la contraction du ventricule. En revanche, si l'on se place du point de vue du flux sanguin pompé par le myocarde, la contraction de l'oreillette (correspondant à la composante EA4) achève le remplissage du ventricule en fin de diastole (composante EA2) et se situe donc, du point de vue de l'hémodynamique cardiaque, après cette dernière - d'où la désignation "PEA4".

La Figure 2 illustre les différents signaux caractérisant l'activité du coeur au cours d'un cycle cardiaque, avec : le profil des pressions intracardiaques, un relevé d'électrocardiogramme de surface (ECG), et les variations du signal d'accélération endocardiaque (EA).

Sur le profil des pressions intracardiaques, la caractéristique P_{A} illustre les variations de la pression aortique, P_{VG} celles du ventricule gauche et P_{OG} celles dans l'oreillette gauche. Les points A à E correspondent aux différentes phases suivantes : A, contraction de l'oreillette gauche ; B, fermeture de la valvule mitrale ; C, ouverture de la valvule aortique ; D, fermeture de la valvule aortique ; et E, ouverture de la valvule mitrale.

Le signal ECG comprend successivement : l'onde P correspondant à la dépolarisation de l'oreillette, le complexe QRS correspondant à la dépolarisation du ventricule et l'onde T de repolarisation ventriculaire.

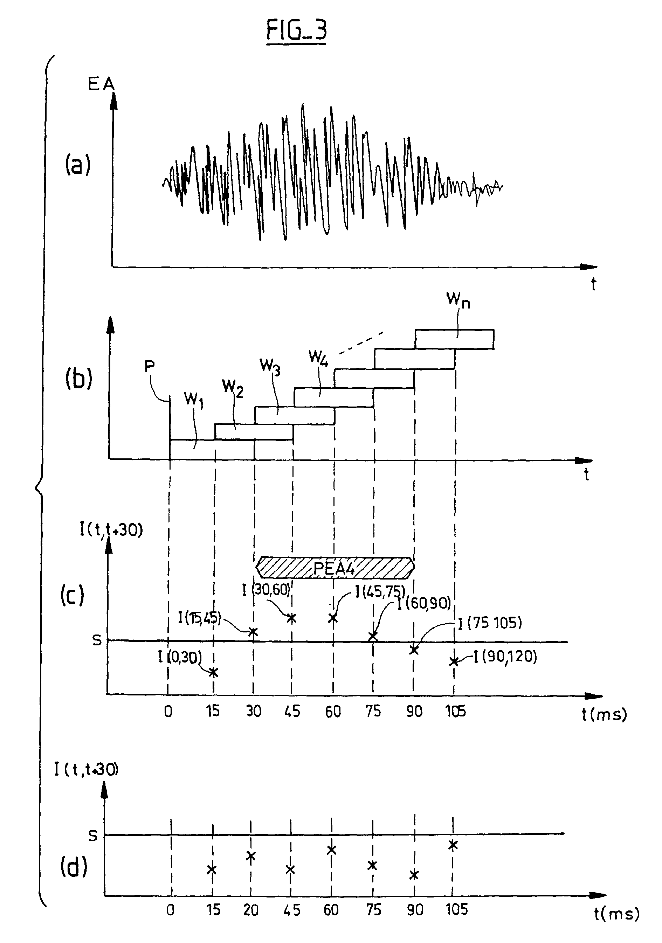

Le signal d'accélération endocardiaque EA, quant à lui, peut se décomposer de la manière suivante : la composante EA4 correspond à la contraction de l'oreillette (onde P), et elle est suivie par la composante EA1, qui débute à la suite du complexe QRS et est engendrée par une combinaison de la fermeture des valves auriculo-ventriculaires, de l'ouverture des valves semi-lunaires et de la contraction du ventricule gauche. La composante EA2 qui lui fait suite, quant à elle, accompagne la fin de la systole ventriculaire et elle est générée par la fermeture des valves semi-lunaires. La Figure 3 illustre, sous forme d'une série de chronogrammes, un exemple avantageux de mise en oeuvre de la présente invention pour la détection de la composante EA4, permettant notamment (i) de détecter la présence ou non d'un pic PEA4 et (ii) dans l'affirmative, de déterminer les instants de début et de fin de ce pic.

Le chronogramme de la Figure 3a illustre le signal d'accélération endocardiaque EA dans la période qui suit immédiatement la stimulation auriculaire, événement indiqué par le marqueur P sur le chronogramme de la Figure 3b.

Cet événement P déclenche une première fenêtre temporelle W1 (Figure 3b), par exemple d'une durée de 30 ms. Sur la durée de cette fenêtre est calculé un indice I(0,30) représentatif de la puissance du signal EA sur l'intervalle 0-30 ms. Cet indice est par exemple déterminé à partir des valeurs numérisées du signal échantillonné, en calculant l'intégrale de la valeur absolue du ce signal EA sur cet intervalle.

La valeur de l'indice I(0,30) qui représente donc la puissance moyenne du signal EA sur la durée de la fenêtre W1 centrée sur l'instant t = 15 ms, est reportée par un point représenté sur le chronogramme de la Figure 3c à l'abscisse t = 15 ms.

Cette détermination est réitérée pour une nouvelle fenêtre W2, décalée par rapport à la fenêtre W1 par exemple de 15 ms (le décalage t = 15 ms correspondant à la durée d'une fenêtre, divisée par deux). Cette seconde détermination, effectuée donc sur l'intervalle t = 15-45 ms, donne un nouvel indice I(15,45).

La détermination est à nouveau réitérée, sur une succession de fenêtres glissantes W3, W4, ... Wn, chacune décalée de 15 ms par rapport à la fenêtre précédente.

La réitération est poursuivie par exemple jusqu'à détection d'un événement ventriculaire, ou jusqu'à ce que l'indice I(t,t+30) passe en dessous d'un seuil limite, ou encore après écoulement d'une période fixe limite, typiquement d'une centaine de millisecondes.

Sur la base de l'évaluation que l'on vient de décrire, on considère qu'il y a contraction auriculaire sur le cycle cardiaque en question si l'indice I(t,t+30) est, pour au moins l'une des fenêtres, supérieur à un seuil prédéterminé S (Figure 3c).

La définition de ce seuil S peut être arbitraire, ou adaptée au patient (paramétrable par le praticien) ou bien encore résulter d'un calcul adaptatif remis à jour régulièrement. Comme exemple particulier de seuil adaptatif, on peut considérer un événement auriculaire spontané (dépolarisation non stimulée de l'oreillette) et calculer les indices I(0,30), I(15,45), I(30,60), ...sur une période donnée. Le seuil est ensuite défini comme valant 50 % de la valeur maximale de toutes les valeurs d'indice ainsi calculées. Le seuil peut être recalculé à intervalles réguliers, typiquement une fois par jour, ou bien sur chaque événement auriculaire validé par le dispositif.

Si le test ainsi effectué permet d'avérer la présence d'une contraction auriculaire, le dispositif détermine les instants de début et de fin du pic d'accélération endocardiaque de la composante EA4 (PEA4), par exemple en considérant que le pic s'étend de la première valeur dépassant le seuil jusqu'à la dernière valeur encore située au-dessus de ce même seuil, comme illustré sur la Figure 3c.

On a ainsi déterminé la présence d'un pic PEA4 - donc l'existence d'une contraction de l'oreillette en réponse à la stimulation auriculaire - et les instants de début et de fin de ce pic PEA4.

Cette détermination peut notamment être appliquée à un test de capture auriculaire : le dispositif applique une stimulation auriculaire à énergie prédéfinie, puis teste la présence de la réponse évoquée de l'oreillette en recherchant un pic PEA4 de la manière que l'on vient d'exposer; ce pic PEA4 révélant l'existence d'une contraction auriculaire suite à la stimulation.

Le test de capture auriculaire peut avantageusement être exploité dans le cadre d'une recherche de seuil de stimulation auriculaire.

À cet effet, le dispositif applique à l'oreillette des impulsions stimulations à énergie décroissante et contrôle à chaque fois sur le signal EA la présence ou non d'une composante EA4 (pic PEA4) par la méthode précédemment décrite. Si la composante PEA4 est effectivement présente (c'est-à-dire, dans l'exemple décrit plus haut, si l'indice I(t,t+30) est supérieur au seuil S sur l'une au moins des fenêtres glissantes W1, W2, ... , Wn), le dispositif considère que la stimulation auriculaire est une stimulation efficace. L'énergie appliquée pour la stimulation auriculaire suivante est réduite, typiquement d'un pas d'amplitude fixe, par exemple de 0,25 V. Dès que l'indice I(t,t+30) devient toujours inférieur au seuil S (situation illustré sur la Figure 3d), alors le dispositif considère que la stimulation est inefficace, et donc que le seuil de stimulation auriculaire est supérieur à la dernière valeur appliquée. Dans ce dernier cas, une stimulation de sécurité à amplitude maximale peut être appliquée afin de provoquer en tout état de cause une contraction auriculaire.

Le seuil de stimulation auriculaire ainsi déterminé peut être conservé dans les mémoires de l'appareil, être transmis à un centre de recueil de données, ou encore utilisé par l'implant pour modifier l'amplitude de stimulation appliquée.

Pour d'autres détails sur les algorithmes d'ajustement de l'amplitude de stimulation à partir de tests de capture successifs, on pourra se référer notamment au EP 1 080 744 A1 (ELA Medical), qui décrit diverses techniques de mesure du seuil, de contrôle de cohérence des mesures et d'ajustement de la largeur et de l'amplitude de l'impulsion de stimulation. Les algorithmes correspondants peuvent être mis en oeuvre en utilisant, en lieu et place d'une détection électrique de la dépolarisation de l'oreillette, le test de capture effectué selon les enseignements de l'invention, c'est-à-dire par analyse du signal EA et recherche de la présence d'une composante EA4.

## Revendications

1. Un dispositif médical actif implantable du type prothèse cardiaque de stimulation, resynchronisation et/ou défibrillation, comportant :
- des moyens de stimulation auriculaire, aptes à délivrer des impulsions de stimulation auriculaire (P) de faible énergie destinées à être appliquées à une électrode implantée dans la cavité auriculaire d'un patient ; et
- des moyens de test de capture auriculaire, aptes à détecter la survenue d'une contraction auriculaire consécutive à l'application d'une impulsion de stimulation auriculaire, et comprenant un capteur d'accélération, apte à délivrer un signal d'accélération endocardiaque (EA) représentatif des mouvements produits par les contractions cycliques de la cavité auriculaire, et des moyens d'analyse du signal d'accélération endocardiaque (EA),
dispositif **caractérisé en ce que** :
- les moyens d'analyse du signal d'accélération endocardiaque (EA) sont des moyens aptes à reconnaître et isoler dans le signal d'accélération endocardiaque (EA) délivré par le capteur une composante correspondant au quatrième pic d'accélération endocardiaque (EA4) et associée à la contraction de l'oreillette et suivie (i) d'une composant correspondant au premier pic d'accélération endocardiaque (EA1) et associée au début du complexe QRS et engendrée par une combinaison de la fermeture des valves auriculo-ventriculaires, de l'ouverture des valves semi-lunaires et de la contraction du ventricule gauche, et (ii) d'une composante correspondant au deuxième pic d'accélération endocardiaque (EA2) consécutive, associée à la fin de la systole ventriculaire et générée par la fermeture des valves semi-lunaires, et
- les moyens de test de capture auriculaire sont des moyens aptes à déterminer la présence ou non de ladite composante correspondant au quatrième pic d'accélération endocardiaque (EA4) par quantification d'un paramètre (I(_{t,t,30)}) d'énergie du Signal d'accélération endocardiaque (EA) intégré sur la durée d'une fenêtre temporelle d'analyse (Wᵢ) de durée prédéterminée,
ladite fenêtre d'analyse étant déclenchée après l'instant d'application d'une desdites impulsions de stimulation auriculaire (P) et se terminant sur un autre événement spécifique,
la présence/l'absence de ladite composante correspondant au quatrième pic d'accélération endocardiaque (EA4) étant respectivement représentative d'une capture/d'une perte de capture auriculaire.

2. Le dispositif de la revendication 1, dans lequel ledit autre événement spécifique est un événement du groupe comprenant : détection ventriculaire, stimulation ventriculaire, apparition d'un signal correspondant au premier pic d'accélération endocardiaque (EA), ou délai fixe.

3. Le dispositif de la revendication 1, dans lequel les moyens d'analyse de signal d'accélération endocardiaque (EA) comprennent des moyens pour quantifier le paramètre (I(ₜ,ₜ,₃₀₎) du signal d'accélération endocardiaque (EA) à l'intérieur d'une pluralité de fenêtres temporelles d'analyse successives (W₁ ... Wₙ).

4. Le dispositif de la revendication 3, dans lequel les fenêtres temporelles d'analyse successives (W₁ ... Wₙ) sont des fenêtres chevauchantes.

5. Le dispositif de la revendication 1, dans lequel les moyens d'analyse de signal d'accélération endocardiaque (EA) comprennent des moyens comparateurs, pour comparer à un seuil donné (S) le paramètre (I(_{t,t,30)}) du signal d'accélération endocardiaque (EA) quantifié à l'intérieur de la fenêtre temporelle (Wᵢ).

6. Le dispositif de la revendication 5, dans lequel le seuil (S) est un seuil du groupe formé par : seuil fixe ; seuil paramétrable ; seuil adaptatif recalculé à intervalles réguliers ; seuil adaptatif recalculé sur chaque cycle comprenant un événement auriculaire valide.

7. Le dispositif de la revendication 1, comprenant en outre :
- des moyens de recherche du seuil de capture auriculaire, aptes à modifier de manière itérative l'énergie de l'impulsion de stimulation délivrée par les moyens de stimulation auriculaire, et à tester à chaque fois la capture ou la perte de capture auriculaire.

8. Le dispositif de la revendication 1, dans lequel le capteur d'accélération est un capteur du groupe formé par : capteur endocavitaire ; capteur épicardique ; capteur externe.

## Claims

1. Implantable active medical device of the stimulation, resynchronization and/or defibrillation cardiac prosthesis type, comprising:
- atrial stimulation means, capable of delivering atrial stimulation pulses (P) of low energy intended to be applied to an electrode implanted in the atrial cavity of a patient; and
- atrial capture testing means, capable of detecting the occurrence of an atrial contraction following the application of an atrial stimulation pulse and comprising an acceleration sensor, capable of delivering an endocardiac acceleration signal (EA) representative of the movements produced by the cyclic contractions of the atrial cavity, and means for analysing the endocardiac acceleration signal (EA),
the device being **characterized in that**:
- the means for analysing the endocardiac acceleration signal (EA) are means capable of recognizing and isolating, in the endocardiac acceleration signal (EA) delivered by the sensor, a component corresponding to the fourth endocardiac acceleration spike (EA4) and associated with the contraction of the atrium and followed (i) by a component corresponding to the first endocardiac acceleration spike (EA1) and associated with the start of the QRS complex and generated by a combination of the closure of the atrial-ventricular valves, with the opening of the semi-lunar valves and the contraction of the left ventrical, and (ii) by a consecutive component corresponding to the second endocardiac acceleration spike (EA2), associated with the end of the ventricular systole and generated by the closure of the semi-lunar valves, and
- the atrial capture testing means are means capable of determining the presence or absence of said component corresponding to the fourth endocardiac acceleration spike (EA4) by quantization of an energy parameter (1_{(t,t+30)}) of the endocardiac acceleration signal (EA) integrated over the duration of an analysis time window (Wᵢ) of predetermined duration,
said analysis window being triggered after the instant of application of one of said atrial stimulation pulses (P) and ending on another specific event,
the presence/absence of said component corresponding to the fourth endocardiac acceleration spike (EA4) being respectively representative of an atrial capture/loss of capture.

2. Device according to Claim 1, in which said other specific event is an event from the group comprising: ventricular detection, ventricular stimulation, appearance of a signal corresponding to the first endocardiac acceleration spike (EA1), or fixed delay.

3. Device according to Claim 1, in which the means for analysing the endocardiac acceleration signal (EA) comprise means for quantizing the parameter (1_{(t,t+30)}) of the endocardiac acceleration signal (EA) within a plurality of successive analysis time windows (W_{1...}Wₙ).

4. Device according to Claim 3, in which the successive analysis time windows (W_{1...}Wₙ) are overlapping windows.

5. Device according to Claim 1, in which the means for analysing the endocardiac acceleration signal (EA) comprise comparator means, for comparing the parameter (1_{(t,t+30)}) of the endocardiac acceleration signal (EA) quantized within the time window (Wᵢ) with a given threshold (S).

6. Device according to Claim 5, in which the threshold (S) is a threshold from the group formed by: fixed threshold; configurable threshold; adaptive threshold recalculated at regular intervals; adaptive threshold recalculated on each cycle comprising a valid atrial event.

7. Device according to Claim 1, further comprising:
- means for searching for the atrial capture threshold, capable of iteratively modifying the energy of the stimulation pulse delivered by the atrial stimulation means, and for each time testing the atrial capture or loss of capture.

8. Device according to Claim 1, in which the acceleration sensor is a sensor from the group formed by: endocavitary sensor; epicardiac sensor; external sensor.

## Patentansprüche

1. Implantierbare aktive medizinische Vorrichtung des Typs Stimulations-, Resynchronisations- und/oder Defibrillations-Herzprothese, die Folgendes umfasst:
- atriale Stimulationsmittel, die dazu ausgelegt sind, atriale Stimulationsimpulse (P) mit geringer Energie zu verabreichen, die dazu bestimmt sind, an eine im Atrium eines Patienten implantierte Elektrode angelegt zu werden; und
- Atriumsensor-Testmittel, die dazu ausgelegt sind, das Auftreten einer atrialen Kontraktion als Folge des Anlegens eines atrialen Stimulationsimpulses zu detektieren, und einen Beschleunigungssensor, der dazu ausgelegt ist, ein endokardiales Beschleunigungssignal (EA) zu liefern, das die durch die zyklischen Kontraktionen des Atriums erzeugten Bewegungen repräsentiert, sowie Mittel zur Analyse des endokardialen Beschleunigungssignals (EA) umfassen,
wobei die Vorrichtung **dadurch gekennzeichnet ist, dass**:
- die Mittel zum Analysieren des endokardialen Beschleunigungssignals (EA) Mittel sind, die dazu ausgelegt sind, in dem endokardialen Beschleunigungssignal (EA), das von dem Sensor geliefert wird, eine Komponente zu erkennen und zu isolieren, die der vierten endokardialen Beschleunigungsspitze (EA4) entspricht und der Kontraktion des Herzohrs zugeordnet ist und der (i) eine Komponente, die der ersten Spitze der endokardialen Beschleunigung (EA1) entspricht und die dem Beginn des QRS-Komplexes zugeordnet ist und durch eine Kombination aus dem Schließen der atrial-ventrikulären Klappen und dem Öffnen der semilunaren Klappen und der Kontraktion des linken Ventrikels erzeugt wird, und (ii) eine darauf folgende Komponente, die der zweiten Spitze der endokardialen Beschleunigung (EA2) entspricht und die dem Ende der ventrikulären Systole zugeordnet ist und durch das Schließen der semilunaren Klappen erzeugt wird, folgen, und
- die Atriumsensor-Testmittel Mittel sind, die dazu ausgelegt sind, das Vorhandensein oder Fehlen der Komponente, die der vierten Spitze der endokardialen Beschleunigung (EA4) entspricht, durch Quantifizieren eines Parameters (1_{(t,t+30)}) der Energie des endokardialen Beschleunigungssignals (EA), das über die Dauer eines Analysezeitfensters (Wᵢ) mit vorgegebener Dauer integriert wird, zu bestimmen,
wobei das Analysefenster nach dem Zeitpunkt des Anlegens eines der atrialen Stimulationsimpulse (P) beginnt und bei einem weiteren spezifischen Ereignis endet,
wobei das Vorhandensein/Fehlen der Komponente, die der vierten Spitze der endokardialen Beschleunigung (EA4) entspricht, einen/keinen Einfang des Atriumsensors repräsentiert.

2. Vorrichtung nach Anspruch 1, wobei das weitere spezifische Ereignis ein Ereignis aus der Gruppe ist, die Folgendes umfasst: ventrikuläre Detektion, ventrikuläre Stimulation, Auftreten eines Signals, das der ersten Spitze der endokardialen Beschleunigung (EA1) entspricht, oder feste Verzögerung.

3. Vorrichtung nach Anspruch 1, wobei die Mittel zur Anlayse des endokardialen Beschleunigungssignals (EA) Mittel umfassen, um den Parameter (1(_{t,t+30)}) des endokardialen Beschleunigungssignals (EA) in mehreren aufeinander folgenden Analysezeitfenstern (W₁, ..., Wₙ) zu quantifizieren.

4. Vorrichtung nach Anspruch 3, wobei die aufeinander folgenden Analysezeitfenster (W₁, ..., Wₙ) überlappende Fenster sind.

5. Vorrichtung nach Anspruch 1, wobei die Mittel zur Analyse des endokardialen Beschleunigungssignals (EA) Komparatormittel umfassen, um den Parameter (1_{(t,t+30)}) des endokardialen Beschleunigungssignals (EA), das innerhalb des Zeitfensters (Wᵢ) quantifiziert ist, mit einem vorgegebenen Schwellenwert (S) zu vergleichen.

6. Vorrichtung nach Anspruch 5, wobei der Schwellenwert (S) ein Schwellenwert der Gruppe ist, die Folgendes umfasst: festen Schwellenwert; parametrisierbaren Schwellenwert; adaptiven Schwellenwert, der in regelmäβigen Intervallen neu berechnet wird; adaptiven Schwellenwert, der in jedem Zyklus, der ein gültiges atriales Ereignis enthält, neu berechnet wird.

7. Vorrichtung nach Anspruch 1, die außerdem Folgendes umfasst:
- Mittel zum Suchen des Atriumsensor-Schwellenwerts, die dazu ausgelegt sind, die Energie des durch die atrialen Stimulationsmittel verabreichten Stimulationsimpulses iterativ zu modifizieren und jedesmal den Einfang oder den Nichteinfang des Atriumsensors zu testen.

8. Vorrichtung nach Anspruch 1, wobei der Beschleunigungssensor ein Sensor aus der Gruppe ist, die Folgendes umfasst: endokavitären Sensor; epikardialen Sensor; externen Sensor.
